# EUROPEAN PATENT APPLICATION

(11) **EP 1 884 525 A1**
(43) Date of publication of application: **06.02.2008**
(21) Application number: 06291272.0
(22) Date of filing: 03.08.2006
(51) Int. Cl.: C08F 4/622, C08F 4/70, C08F 10/02, C07C 251/70

(54) **Polymerisation catalyst system based on oxime-ether ligands**

(71) Applicant: TOTAL PETROCHEMICALS RESEARCH FELUY, 7181 Seneffe (Feluy) (BE); Centre National de la Recherche Scientifique (CNRS), 75016 Paris (FR)
(72) Inventor: Boulanger, Loise, 35690 Acigné (BE); Lavastre, Olivier, 35490 Gahard (FR); Sirol, Sabine, 1440 Wauthier-Brain (BE); Razavi, Abbas, 7000 Mons (BE)
(74) Representative: Roufosse, Micheline C.

(57) **Abstract**

The present invention discloses metallic complexes based on oxime-ether ligand and their use in oligomerisation and in polymerisation of ethylene and alpha-olefins.

## Description

This invention relates to the field of oxime-ether ligands and their use in catalyst system for the polymerisation and oligomerisation of ethylene and alpha-olefins.

There exists a multitude of catalyst systems available for polymerising or oligomerising ethylene and alpha-olefins, but there is a growing need for finding new systems capable to tailor polymers with very specific properties. More and more post-metallocene catalyst components based on early or late transition metals from Groups 3 to 10 of the Periodic Table have recently been investigated such as for example those disclosed in Gibson and al. review (Gibson, V.C.; Spitzmesser, S.K., Chem. Rev. 2003, 103, p. 283). But there is still a need to improve either the specificities or the performances of these systems.

It is an aim of the present invention to prepare a polymerisation catalyst system based on oxime-ether ligands.

It is also an aim of the present invention to use oxime-ether ligand-based catalyst system for the homo- or co-polymerisation of ethylene and alpha-olefins.

Accordingly, the present invention discloses oxime-ether ligands of general formula I 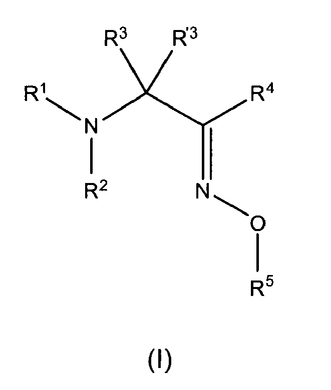 wherein wherein R¹, R², R³, R'³ and R⁴ are each independently selected from hydrogen or alkyl group having from 1 to 20 carbon atoms, or aryl group having from 3 to 18 carbon atoms or heterocycles or wherein two neighbouring R¹ can be linked together to form a ring and
wherein R⁵ is a alkyl, benzyl or phenyl compound of formula 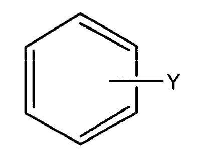 wherein there is at least one substituent Y on the phenyl group, and each Y is the same or different and at least one Y is an electron attracting group.

The other Y substituents, if present, can be steric substituents such as for example alkyl or aryl groups.

Preferably, the electron attracting group Y is NO₂ or CN.

Among the preferred embodiments according to the present invention, R¹ and R² can each be independently selected from isopropyl, n-butyl, benzyl, cyclohexyl, pyridyl, méthylpyridine, thiényl, thényl, furyl, furfuryl, phenyl, mesityl. R³ and R'³ are preferably hydrogen and R⁴ is preferably an alkyl group having from 1 to 6 carbon atoms, more preferably methyl.

The invention also discloses a process for preparing an oxime-ether ligand that comprises the steps of:
a) providing an oxime ligand of formula II 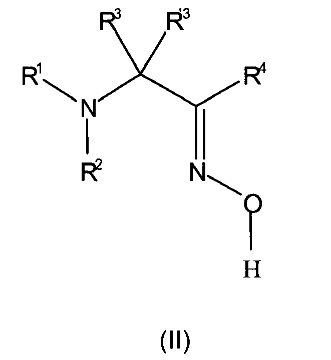 wherein R¹, R², R³, R'³ and R⁴ are each independently selected from H or alkyl groups having from 1 to 20 carbon atoms or aryl groups having from 3 to 18 carbon atoms or functional groups such as heterocycles or two neighbouring Rⁱ can be linked together to form a ring.
b) deprotonating the OH group of the oxime ligand in the presence of a base;
c) in the presence of a polar solvent; and
d) optionally in the presence of a crown ether able to trap the cation from the base of step b).
e) reacting the anion obtained from steps b), c) and d) with R⁵-X wherein X is a halogen, and R⁵ is alkyl or aryl, preferably R⁵-X is BnBr where Bn is benzyl, or a phenyl group carrying a fluor substitutent and at least one other substituent Y that is an electron attracting group.

The preparation of the ligand can typically be represented by the following scheme. 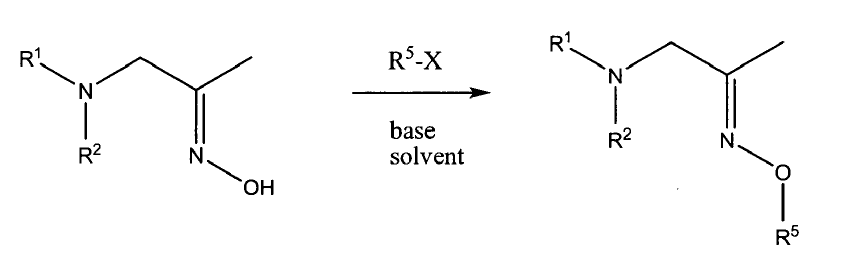 wherein R¹, R² and R⁵ are as described hereabove.

The catalyst component is then prepared by complexing the ligand with a metallic precursor MXᵥ in a ligand to metal ratio of from 1/1 to 2/1. The metallic precursor and the ligand are placed in a solvent and they are allowed to react under stirring for a period of time of from 2 to 10 hours at a temperature of from 10 to 80 °C preferably at room temperature (about 25 °C).

Metal M is selected from groups 6 to 10 of the Periodic Table. Preferably, it is Cr, Fe, Co, Ni, Pd, more preferably it is nickel. X is halogen and v is the valence of M.

The solvent is polar or apolar. Preferably it is tetrahydrofuran (THF).

An active catalyst system is then prepared by adding an activating agent having an ionising action.

Any activating agent having an ionising action known in the art may be used for activating the monooxime catalyst component. For example, it can be selected from aluminium-containing or boron-containing compounds. The aluminium-containing compounds comprise aluminoxane and/or alkyl aluminium.

The aluminoxanes are preferred and may comprise oligomeric linear and/or cyclic alkyl aluminoxanes represented by the formula: 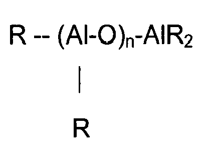 for oligomeric, linear aluminoxanes and 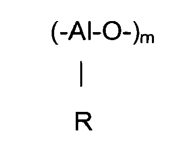 for oligomeric, cyclic aluminoxane,
wherein n is 1-40, preferably 10-20, m is 3-40, preferably 3-20 and R is a C₁-C₈ alkyl group and preferably methyl.

Suitable boron-containing activating agents that can be used comprise a triphenylcarbenium boronate such as tetrakis-pentafluorophenyl-boratotriphenylcarbenium as described in EP-A-0427696, or those of the general formula [L'-H] + [B Ar₁ Ar₂ X₃ X₄]- as described in EP-A-0277004 (page 6, line 30 to page 7, line 7).

The preferred activating agent is aluminoxane. The amount of aluminoxane necessary to activate the catalyst component is selected to have a AI/M ratio of from 100 to 3000, preferably about 1000.

The catalyst system can also be supported. The support if present can be a porous mineral oxide, advantageously selected from silica, alumina and mixtures thereof. Preferably it is silica.

The present invention also discloses a method for oligomerising and for homo- or copolymerising ethylene and alpha-olefins that comprises the steps of:
a) injecting the active catalyst system into the reactor;
b) injecting the monomer and optional comonomer into the reactor;
c) maintaining under polymerising conditions;
d) retrieving the oligomers and polymers.

The polymerisation and oligomerisation method is not particularly limited and it can be carried out at a temperature of from 20 to 85°C and under a pressure of from 0.5 to 50 bars. Preferably, the pressure is of at least 15 bars, more preferably of at least 20 bars.

The preferred monomers and comonomers are selected from ethylene, propylene and hexene.

### List of figures.

Figure 1 represents the molecular weight distribution of the polymer obtained with a catalyst system based on metallic complex C1.

Figure 2 represents the molecular weight distribution of the polymer obtained with a catalyst system based on metallic complex C2.

Figure 3 represents the molecular weight distribution of the polymer obtained with a catalyst system based on metallic complex C3.

Figure 4 represents the molecular weight distribution of the polymer obtained with a catalyst system based on metallic complex C4.

### Examples.

### 1. Synthesis of ligand L1 - benzyl-furan-2-ylmethyl-amino)-propan-2-one 0-benzyl-oxime.

In a balloon containing 1.75 mmol of oxime ligand, 25 mL of dimethylformamide (DMF) were added, then 1.75 mmol of NaH. The mixture was kept under stirring for a period of time of one hour. 1.75 mmol of BnBr were then added and stirring was maintained for a period of time of 20 hours. The solvent was vaporised and the mixture was purified by silica gel chromatography to obtain the resulting oxime-ether with a yield of 91 % as a pale yellow oil.

The following scheme was used:

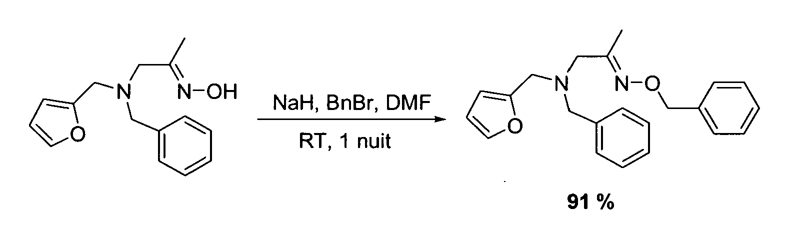

The compound was characterised by NMR.
**RMN ¹H** (300 MHz, CDCl₃) □ : 7.28-7.40 (m, 11 H), 6.34 (m, 1 H), 6.17 (m, 1 H), 5.13 (s, 2H), 3.60 (s, 2H), 3.58 (s, 2H), 3.13 (s, 2H), 1.96 (s, 3H);
**RMN ¹³C** (75 MHz, COCl₃) □ : 157.1, 152.2, 142.0, 138.9, 138.3, 128.9, 128.3, 128.2, 127.8, 127.6, 127.0, 110.1, 108.9, 75.4, 57.4, 57.3, 49.5, 13.1;
**EIMS** *m*/*z* [M-CH₂C₄H₃O]⁺ 267.1498, calcd for C₁₇H₁₉N₂O 267.1497; Anal. Calcd C, 75.83; H, 6.94; N, 8.04. Found: C, 75.83; H, 6.89; N, 8.02

### 2. Synthesis of arvl-substituted oxime-ether ligands.

The introduction of a simple phenyl group was impossible for lack of reactivity of fluorobenzene. Fluorinated derivatives activated with electro-attracting substituents were selected.
The general procedure was the same as that used for the preparation of O-benzyle oxime-ether ligands and the following scheme was used.

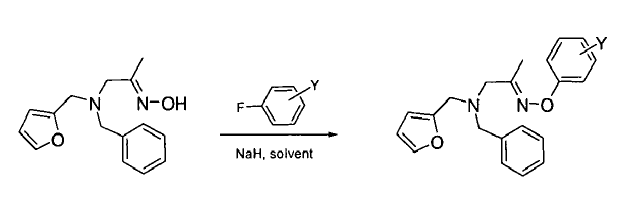

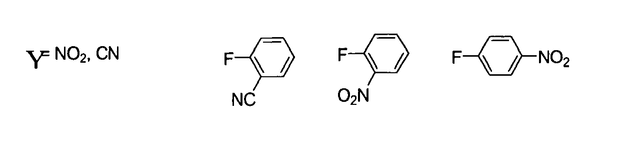

Specific amounts of reactants and solvents were selected according to each substituted fluorobenzene.

### Ligand L2 - (1-benzyl-furan-2-ylmethyl-amino)-propan-2-one 0-(2-nitro-phenyl)-oxime.

For Y = NO₂ 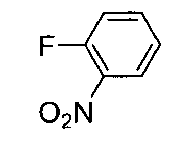 1.5 equivalents of fluorinated derivative substrate were used for 1 equivalent of NaH and 1 equivalent of oxime. The solvent was tetrahydrofurane (THF) 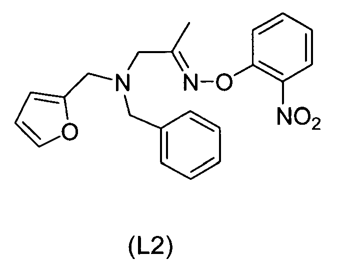 was obtained with a yield of 87 % as a yellow oil.

The compound was characterised by NMR.
**RMN ¹H** (300 MHz, CDCl₃) □ : 7.97 (dd, *J₁* = 1.5 Hz, *J₂* = 8.3 Hz, 1H), 7.74 (dd, *J₁ =* 1.1 Hz, *J₂* = 8.3 Hz, 1 H), 7.57 (td, *J₁* = 1.5 Hz, *J₂* = 7.9 Hz, 1 H), 7.26-7.42 (m, 7H), 7.06 (td, *J₁* = 1.5 Hz, *J₂* = 7.9 Hz, 1 H), 6.35 (dd, *J₁* = 1.9 Hz, *J₂* = 3.4 Hz, 1 H), 6.23 (d, J = 3.0 Hz, 1 H), 3.67 (s, 2H), 3.64 (s, 2H), 3.27 (s, 2H), 2.17 (s, 3H);
**RMN ¹³C** (75 MHz**,** CDCl₃) □ : 163.6, 153.1, 151.9, 142.2, 138.5, 137.3, 134.5, 129.0, 128.9, 128.8, 128.4, 127.3, 125.5, 121.2, 117.1, 110.2, 109.1, 57.8, 56.7, 49.8, 14.3;
**EIMS** *m*/*z* [M-CH₂C₄H₃O]⁺ 298.1197, calcd for C₁₆H₁₆N₃O₃ 298.1192; **Anal.** Calcd C, 66.48; H, 5.58; N, 11.07. Found: C, 67.22; H, 6.04; N, 10.32.

### Or

### Ligand L3 - 1-(benzyl-furan-2-ylmethyl-amino)-propan-2-one 0-(4-nitro-phenyl)-oxime.

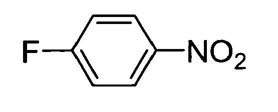

1 equivalent of fluorinated derivative substrate was used for 1 equivalent of NaH and 1 equivalent of oxime. The solvent was DMF. 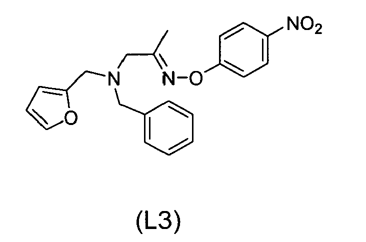 was obtained with a yield of 60 % as an orange oil.
NMR results were as follows.
**RMN ¹H** (300 MHz, CDCl₃) □ : 8. 21 (m, 2H), 7.25-7.39 (m, 8H), 6.36 (dd, *J₁* = 1.9 Hz, *J₂* = 3.0 Hz, 1 H), 6.24 (d, J = 3.0 Hz, 1 H), 3.68 (s, 2H), 3.66 (s, 2H), 3.29 (s, 2H), 2.11 (s, 3H);
**RMN ¹³C** (75 MHz, COCl₃) □ : 164.1, 162.8, 151.8, 142.2, 138.5, 128.9, 128.4, 127.3, 125.7,114.3, 110.2, 109.1, 57.8, 56.9, 49.9, 13.8;
**EIMS** *m*/*z* [M-CH₂Ph]⁺ 288.0991, calcd for C₁₄H₁₄N₃O₄ 288.0984; **Anal.** Calcd C, 66.48; H, 5.58; N, 11.07. Found: C, 66.97; H, 5.61; N, 11.03.

### Ligand L4 - 2-[2-(benzyl-furan-2-ylmethyl-amino)-1-methyl-ethylideneaminooxy]-benzonitrile

### For Y = CN

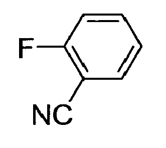

1.5 equivalents of fluorinated derivative substrate were used for 1 equivalent of NaH and 1 equivalent of oxime. The solvent was DMF. 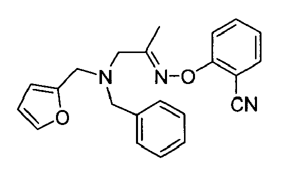 was obtained with a yield of 68 % as a pale yellow oil.

NMR results were as follows.
**RMN ¹H** (300 MHz**,** CDCl₃) □ : 7.52-7.56 (m, 3H), 7.26-7.42 (m, 7H), 7.01-7.06 (m, 1 H), 6.35 (dd, *J₁* = 1.9 Hz, *J₂* = 3.4 Hz, 1 H), 6.23 (d, *J* = 3.0 Hz, 1 H), 3.68 (s, 2H), 3.65 (s, 2H), 3.28 (s, 2H), 2.17 (s, 3H);
**RMN ¹³C** (75 MHz, CDCl₃) □ : 163.1, 161.0, 151.9, 142.2, 138.5, 134.3, 133.0, 128.9, 128.4, 127.3, 121.8, 116.0, 114.9, 110.2, 109.1, 99.4, 57.8, 56.7, 49.8, 13.9;
**EIMS** *m*/*z* [M]⁺ 359.1638, calcd for C₂₂H₂₁N₃O₂ 359.1634; **Anal.** Calcd C, 73.52; H, 5.89; N, 11.69. Found: C, 73.47; H, 5.91; N, 11.66.

### Synthesis of chromium complexes.

In a glove box, CrCl₂ was introduced in a Schlenk and a solution of the ligand in THF was added: the metal concentration was of 3.10⁻² mol/L. The complexation reaction was carried out for a period of time of 6 hours under stirring and then THF was eliminated under vacuum. The solid residue was washed three times with ether in order to eliminate all residual ligand and then dried under vacuum.

The amounts of ligand and metallic complex are summarised in Table I.

**TABLE I.**

| Catalyst component | Ligand used | Proportions metal/ligand | Complex colour |
|---|---|---|---|
| C1 | L1 | 1/1.2 | Green |
| C2 | L2 | 1/1 | Brown |
| C3 | L3 | 1/1.2 | Brown |
| C4 | L4 | 1/1.2 | Green |

### Polymerisation of ethylene.

All catalyst components were activated with methylaluminoxane (MAO) with a ratio Al/Cr of 1000, the solvent was toluene, the polymerisation temperature was of 35 °C and the ethylene pressure was of 15 bars.

The metallic complex was added to a MAO solution (30% in toluene, 730 equ.), and the mixture was stirred for a period of time of 5 to 10 minutes. In the reactor under inert atmosphere were successively added 50 mL of toluene, a scavenger solution consisting of MAO (30% , 270 equ.), completed to 5 mL with toluene and the solution of activated metallic complex. The temperature was increased to its target value of 35 °C and the ethylene pressure was increased to 15 bars. These conditions were maintained during the reaction time of one hour.

After degassing, the oligomers and polymers were retrieved. The polymer was washed with MeOH/HCl 5% then with MeOH and finally with acetone. It was then dried under vacuum overnight. The results are reported in Table II.

**TABLE II.**

| Metal complex | Cata µmol | Activity KgPE/molCr/h | Consumption KgC₂H₄/molCr/h | Polymer/oligomer |
|---|---|---|---|---|
| C1 | 8.6 | 31 | 2187 | 1/70 |
| C2 | 9.4 | 79 | 1960 | 1/25 |
| C3 | 9.6 | 32 | 1790 | 1/56 |
| C4 | 10.2 | 100 | 1356 | 1/14 |

The activity is measured with respect to the polymer production.
The ethylene consumption curves showed very little decrease.

It can be seen that the catalytic systems of the present invention produce simultaneously oligomers and polymers, with a predominance for oligomers. Several catalytic species are thus probably simultaneously present.

Ethylene consumption for all complexes was high. In ethylene polymerisation, ligand C4, functionalised with a cyano group, was the most active and gave the highest polymer/oligomer ratio.

The oligomer analysis carried out by gas chromatography showed predominantly the formation of alpha-oligomers, with a proportion of up to 95 %. In addition, all systems had a similar Shultz-Flory type oligomer distribution up to C₂₄. The C₆/(C₄+C₆) ratio was in the range 0.57 to 0.60.

The polymers were studied by Gel permeation Chromatographt (GPC) and by Differential Scanning Calorimetry (DSC). The polidispersity index PI is the ratio Mw/Mn of the weight average molecular weight Mw over the number average molecular weight Mn. Their properties are summarised in Table III.

**TABLE III.**

| Metal complex | Mn kD | Mw kD | PI | Tm °C |
|---|---|---|---|---|
| C1 | 8177 | 301100 | 36.8 | 125.0 |
| C2 | 8770 | 558768 | 63.7 | 126.9 |
| C3 | 10464 | 646838 | 61.8 | 114.7 |
| C4 | 5599 | 148652 | 26.6 | 127.3 |

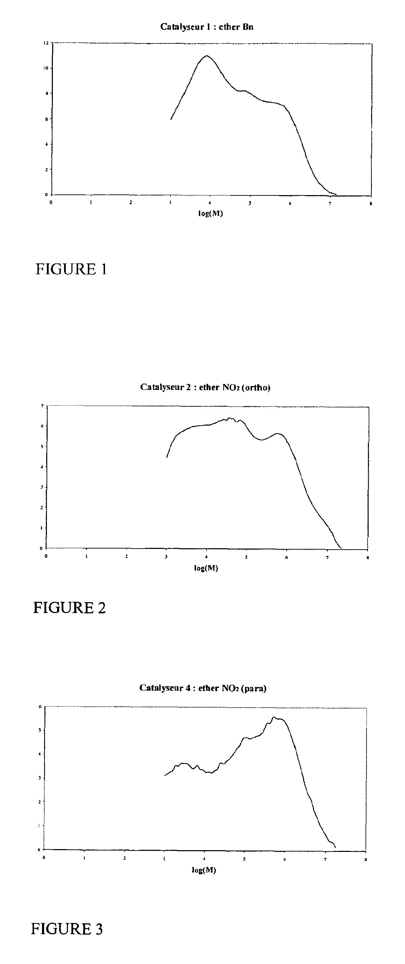

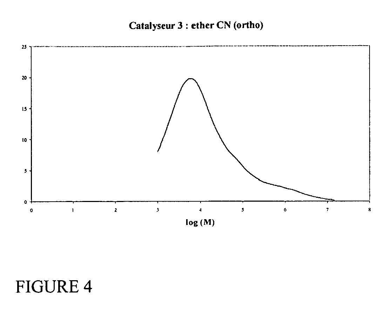

The molecular weight distributions of the polymers produced with catalyst systems based on C1 to C4 are represented respectively in Figures 1 to 4. it can be seen that the polymers prepared with C1 to C3 have a large polydispersity index and a multimodal molecular weight distribution. The polymer formed with complex C4 has a monomodal molecular weight distribution.

The influence of polymerisation temperature and ethylene pressure have been studied for the catalyst systems based on metallic complexes C1 and C4. The results are displayed in Table IV.

**TABLE IV.**

| Metal complex | T °C | P bar | Cr µmol | Activity KgPE/molCr/h | Consump. KgC2/molCr/h | Poly/oligo |
|---|---|---|---|---|---|---|
| C1 | 35 | 15 | 8.6 | 31 | 2187 | 1/70 |
| | 60 | 15 | 10.6 | 20 | 2309 | 1/115 |
| | 35 | 24 | 5.7 | 101 | 2333 | 1 /23 |
| C4 | 35 | 15 | 10.3 | 100 | 1356 | 1/14 |
| | 60 | 15 | 9.3 | 37 | 1914 | 1/52 |
| | 35 | 24 | 9.3 | 299 | 2784 | 1/9 |

It can be seen that for the two catalyst systems studied, an increase in temperature increased the ethylene consumption but reduced the activity in polymer production and the ratio of polymer to oligomer. It was also observed that the stability of the catalyst system decreased with increasing temperature.

Increasing the pressure had a positive impact on all counts: it increased the activity, the ethylene consumption, the polymer to oligomer ratio and the stability.

Preferably, the polymerisation of ethylene should be carried at an ethylene pressure of at least 15 bars, more preferably of at least 20 bars.

## Claims

1. An oxime-ether ligand of formula I 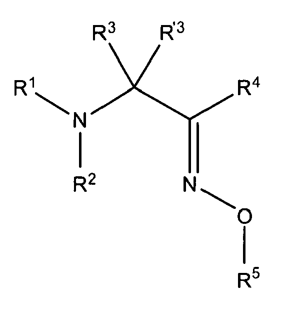 wherein wherein R¹, R², R³, R'³ and R⁴ are each independently selected from hydrogen or alkyl group having from 1 to 20 carbon atoms, or aryl group having from 3 to 18 carbon atoms or heterocycles or wherein two neighbouring Rⁱ can be linked together to form a ring and
wherein R⁵ is a alkyl, benzyl or phenyl compound of formula 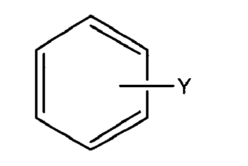 wherein there is at least one substituent Y on the phenyl group, and each additional Y is the same or different and the at least one Y is an electron attracting group.

2. The oxime-ether ligand of claim 1 wherein additional Y substituents are steric substituents selected from alkyl and aryl goups having up to 12 carbon atoms.

3. The oxime-ether ligand of claim 1 or claim 2 wherein R¹ and R² are independently selected from isopropyl, n-butyl, benzyl, cyclohexyl, pyridyl, méthylpyridine, thiényl, thényl, furyl, furfuryl, phenyl or mesityl.

4. The oxime-ether ligand of any one of claims 1 to 3 wherein R³ and R'³ are hydrogen.

5. The oxime-ether ligand of any one of the preceding claims wherein R⁴ is an alkyl group having,from 1 to 6 carbon atoms, preferably methyl.

6. A method for preparing the oxime-ether ligand of any one of claims 1 to 5 that comprises the steps of:
a) providing an oxime ligand of formula II 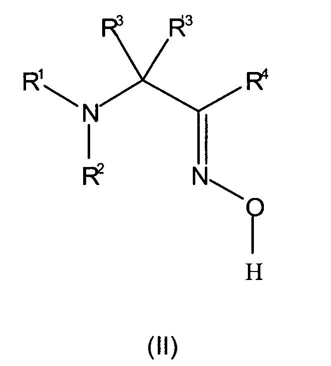 wherein R¹, R², R³, R'³ and R⁴ are each independently selected from H or alkyl groups having from 1 to 20 carbon atoms or aryl groups having from 3 to 18 carbon atoms or functional groups such as heterocycles or two neighbouring Rⁱ can be linked together to form a ring.
b) deprotonating the OH group of the oxime ligand in the presence of a base;
c) in the presence of a polar solvent; and
d) optionally in the presence of a crown ether able to trap the cation from the base of step b).
e) reacting the anion obtained from steps b), c) and d) with R⁵-X wherein X is a halogen, and R⁵ is alkyl or aryl,

7. The process of claim 6 wherein R⁵-X is BnBr where Bn is benzyl.

8. The process of claim 6 wherein R⁵-X is a phenyl group carrying a fluor substitutent and at least one other substituent Y that is an electron attracting group.

9. The process of claim 8 wherein Y is an electron attracting group selected from CN or NO₂.

10. A process for preparing a catalyst component by complexing the oxime-ether ligang of any one of claims 1 to 5 with metallic precursor MXᵥ wherein M is a metal Group 6 to 10 of the Periodic Table, X is halogen and v is the valence of M, and wherein the ratio ligand/metal of 1:1 to 2:1.

11. The process of claim 10 wherein the metal is Cr, Fe, Co, Ni, Pd.

12. A metallic component obtainable by the process of claim 10 or claim 11.

13. An active catalyst system comprising the metallic component of claim 12 and an activating agent having an ionising action.

14. The active catalyst system of claim 13 wherein the activating agent is methylaluminoxane.

15. A process for oligomerising and for homopolymerising ethylene and alpha-olefins that comprises the steps of:
a) injecting the active catalyst system into the reactor;
b) injecting the monomer and optional comonomer into the reactor;
c) maintaining under polymerising conditions;
d) retrieving the oligomers and polymers.

16. The process of claim 15 wherein the monomer is ethylene, propylene or 1-hexene, preferably ethylene.

17. The process of claim 15 or 16 wherein the monomer is ethylene and the ethylene pressure is of at least 20 bars.
